# EUROPÄISCHE PATENTSCHRIFT

(11) **EP 3 553 515 B1**
(45) Veröffentlichungstag und Bekanntmachung des Hinweises auf die Patenterteilung: **23.08.2023**
(21) Anmeldenummer: 19168462.0
(22) Anmeldetag: 10.04.2019
(51) Int. Cl.: G01N 33/14, B01D 3/00, C12H 6/02, G01N 27/06

(54) **VERFAHREN ZUR BESTIMMUNG UND VERARBEITUNG DES NACHLAUFABTRENNZEITPUNKTES BEI DER HERSTELLUNG UND/ODER REINIGUNG VON ALKOHOLISCHEN GETRÄNKEN UND EXTRAKTEN**
METHOD FOR DETERMINING AND PROCESSING THE TAILING SEPARATION TIME IN THE PRODUCTION AND / OR PURIFICATION OF ALCOHOLIC DRINKS AND EXTRACTS
PROCEDE DE DETERMINATION ET DE TRAITEMENT DU TEMPS DE SEPARATION DE SILLAGE LORS DE LA PRODUCTION ET / OU DE LA PURIFICATION DE BOISSONS ALCOOLIQUES ET D'EXTRAITS

(30) Priorität: 11.04.2018 DE 102018108578
(43) Veröffentlichungstag der Anmeldung: 16.10.2019
(73) Patentinhaber: Arnold Holstein GmbH, 88677 Markdorf (DE)
(72) Erfinder: Liebminger, Dr. Andreas, 2130 Paasdorf (AT)
(74) Vertreter: Limbeck, Achim

(56) Entgegenhaltungen:
- DE-U1-202006 005 968
- Christiane Donabaum: "Evaluierung chemischer und physikalischer Parameter zur Nachlaufabtrennung von Stein- und Kernobstdestillaten", Diplomarbeit, Februar 2009 (2009-02), Seiten 1-204, XP055622793, Wien Gefunden im Internet: URL:http://othes.univie.ac.at/3868/1/2009- 02-11_9801105.pdf [gefunden am 2019-09-17]
- MANFRED GÖSSINGER ET AL: "Einfluss verschiedener Prozessparameter auf wichtige Kenngrößen bei der Gleichstromdestillation von Apfelmaische", MITTEILUNGEN KLOSTERNEUBURG, Bd. 61, 2011, Seiten 111-118, XP055622189, DE ISSN: 0007-5922
- GÖSSINGER M., CH. DONABAUM, W. BRANDES, E. BERGHOFER: "Fehlaromen vermeiden - Untersuchungen zur Bestimmung des N-Punktes bei der Obstdestillation", GETRÄNKEINDUSTRIE, vol. 10, 2009, pages 8-11, ISSN: 0016-9323 [retrieved on 2022-01-24]
- LIEBMINGER ANDREAS ET AL: "Automated separation of tail fraction for fruit distillates by means of in-line conductivity measurement", INTERNATIONAL JOURNAL OF FOOD SCIENCE AND TECHNOLOGY , vol. 55, no. 11 15 July 2020 (2020-07-15), pages 3484-3492, XP055882834, GB ISSN: 0950-5423, DOI: 10.1111/ijfs.14682 Retrieved from the Internet: URL:https://onlinelibrary.wiley.com/doi/fu ll-xml/10.1111/ijfs.14682 [retrieved on 2022-01-24]

## Beschreibung

### Technisches Gebiet

Die vorliegende Erfindung betrifft ein Verfahren zur Bestimmung und anschließenden Verarbeitung des Nachlaufabtrennpunktes bei der Herstellung und/oder Reinigung von alkoholischen Getränken oder Extrakten mittels einer Destillationsanlage.

### Stand der Technik

Es ist aus dem Stand der Technik bekannt, dass die Bestimmung des Nachlaufabtrennzeitpunktes (nachstehend auch als sog. N-Punkt bezeichnet) bei der Herstellung von Obstdestillaten in vielen Betrieben ein großes Problem darstellt. Dabei ist die Abtrennung des Nachlaufs von besonderer Bedeutung für die Qualität des herzustellenden Produkts und sollte sorgfältig erfolgen, da durch unzureichend exaktes Abtrennen des Nachlaufs vom Mittellauf entweder vermehrt Fuselalkohole und Fettsäuren in den Mittellauf gelangen, welche die Qualität des Produkts mindern und dieses für den Genuss unzugänglich machen, oder durch zu frühes Umstellen auf den Nachlauf wertvoller Mittellauf und ggf. wichtige Aromabestandteile verloren gehen.

Der N-Punkt wird gemäß dem Stand der Technik häufig durch sensorische Bewertung ermittelt, die physische und psychische Verfassung des Verkosters können jedoch auf sein Kosturteil Einfluss nehmen. Alternativ kann die Bestimmung des N-Punkts über eine empirische Geistrohrtemperatur, oder einen empirischen Alkoholgehalt erfolgen. Die Temperatur des Geistrohres, als auch der Alkoholgehalt variieren jedoch am Umschlagpunkt zwischen Mittellauf und Nachlauf, abhängig vom jeweiligen Brenngerät und dem Alkoholgehalt der Maische (INNERHOFER, G: Das große Buch der Obstverarbeitung, avBuch, Wien (2005)), so dass eine allgemein gültige Festlegung für eine automatische N-Punkt Bestimmung mit diesen Methoden nicht ohne Einschränkungen möglich ist.

Aus der Veröffentlichung von Christiane Donabaum: "Evaluierung chemischer und physikalischer Parameter zur Nachlaufabtrennung von Stein- und Kernobstdestillaten" (Diplomarbeit, Februar 2009 (2009-02), Seiten 1-204, XP055622793, Wien) ist ein Verfahren beschrieben, bei dem die Leitfähigkeit eines Destillats in diskreten Proben, so genannten Fraktionen des Destillats bestimmt wird.

Weiterhin beschreiben Manfred Gössinger et al. sowohl den Einfluss verschiedener Prozessparameter auf wichtige Kenngrößen bei der Gleichstromdestillation von Apfelmaische als auch Untersuchungen zur Bestimmung des N-Punktes bei der Obstdestillation (MITTEILUNGEN KLOS-TERNEUBURG, Bd. 61, 2011, Seiten 111-118, XP055622189, DE, ISSN:0007-5922 sowie Getränkeindustrie 63(10), 2009, Seiten 8-11 ), aus denen hervorging, dass sich Leitfähigkeitsmessungen im allgemeinen Fall bis dato nicht zur Bestimmung des N-Punkes eignen.

In der Veröffentlichung von Andreas Liebminger und Manfred Gössinger et al.: "Automated separation of tail fraction for fruit distillates by means of in-line conductivity measurement " (International Journal of Food Science and Technology, Bd. 55, Nr. 11, 21 Juni 2020 (2020-06-21), Seiten 3484-3492, XP055882834, DOI: 10.1111/ijfs.14682) wurde jedoch die Fähigkeit eines solchen Verfahren zur Bestimmung und Verarbeitung des Nachlaufabtrennpunkts (N) mittels einer kontinuierlicher Messung und Aufzeichnung der elektrischen Leitfähigkeit von Destillaten mittels einer Leitfähigkeitssonde in einer abschließenden Bewertung verifiziert und validiert.

Aus der DE 20 2006 005 968 U1 ist ferner eine Vorrichtung zum Messen einer Alkoholkonzentration einer mit Alkohol gemischten Flüssigkeit bekannt, bei dem objektive physikalische Messparameter zum Umschalten einer Destillationsanlage genutzt werden.

### Darstellung der Erfindung

Der vorliegenden Erfindung liegt die Aufgabe zu Grunde, ein Verfahren zu schaffen, welches die vorgenannten Probleme ausräumt und eine exakt, automatisierte Ermittlung und spätere Verarbeitung des N-Punktes ermöglicht.

Erfindungsgemäß wird die voranstehende Aufgabe gemäß dem Oberbegriff des Anspruchs 1 in Verbindung mit den kennzeichnenden Merkmalen gelöst. Vorteilhafte Ausgestaltungen und Weiterbildungen des erfindungsgemäßen Verfahrens sind in den abhängigen Unteransprüchen angegeben.

Erfindungsgemäß ist ein Verfahren der eingangs genannten Art dadurch gekennzeichnet, dass der Nachlaufabtrennpunkt anhand einer kontinuierlichen Messung und Auswertung der elektrischen Leitfähigkeit des Destillats (vorzugsweise im Auslauf des Kühlers der Destillationsanlage) mittels mindestens einer Leitfähigkeitssonde bestimmt wird, wobei zur Gewährleistung einer automatischen Umschaltung von Mittellauf auf Nachlauf ein mathematischer Algorithmus angewendet wird, welcher in einem Steuergerät bei einem Abweichungswert (z.B. 10% - 25%) von der geringsten während des Mittellaufs gemessenen Leitfähigkeit oder bei einer Änderung der Steigung der Leitfähigkeit dC/dt die Vorlage von Mittellauf auf Nachlauf mechanisch wechselt. Dies kann vorzugsweise über eine Umlenkung des Destillats beim Austritt aus der Vorlage erfolgen, welche vorteilhafterweise entweder über die elektrische oder pneumatische Ansteuerung eines Mehrwegeventils oder durch Drehung der gesamten Vorlage erzielt wird..

Im Nachlauf finden sich vermehrt organische Säuren und deren Ester, sowie ätherische Öle und Wachse, welche die elektrische Leitfähigkeit erhöhen. Da reiner Ethanol praktisch nicht elektrisch leitfähig ist, können geringfügige Mengen an Nachlaufkomponenten mittels kommerziell erhältlicher Leitfähigkeitssonden detektiert werden (Messbereich von 0 - 100 µS/cm).

### Kurzbeschreibung der Zeichnungen

Weitere Ziele, Merkmale, Vorteile und Anwendungsmöglichkeiten des erfindungsgemäßen Verfahrens ergeben sich aus der nachfolgenden Beschreibung eines Ausführungsbeispiels anhand der Zeichnungen. Dabei bilden alle beschriebenen und/oder bildlich dargestellten Merkmale für sich oder in beliebiger Kombination den Gegenstand der Erfindung, unabhängig von der Zusammenfassung in einzelnen Ansprüchen oder deren Rückbeziehung.

In den Zeichnungen zeigen

Fig.1 eine Skizze zur Anbringung der Leitfähigkeitssonde nach dem Kühler der Destillationsanlage;

Fign. 2a, 2b Beispiele einer kontinuierlichen Leitfähigkeitsmessung bei Gleichstromdestillation von zwei verschiedenen Obstmaischen mit unterschiedlichen Alkoholgehalt (EtOH).

### Ausführung der Erfindung

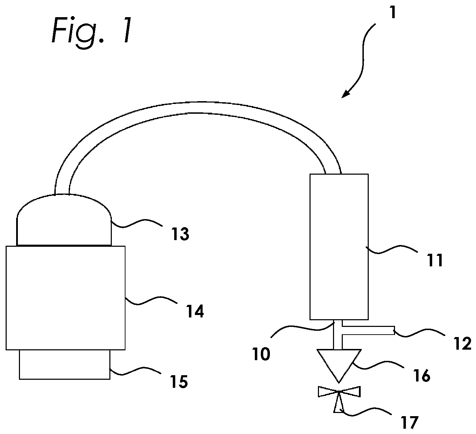

Fig.1 zeigt eine Skizze zur Anbringung der Leitfähigkeitssonde nach dem Kühler 11 einer Gleichstromdestillationsanlage 1. Es sei jedoch klargestellt, dass die vorliegende Erfindung selbstverständlich gleichermaßen auf sämtliche anderen gemäß dem Stand der Technik bekannten Destillationsprozesse und -anlagen sowie unabhängig vom Alkoholgehalt anwendbar ist.

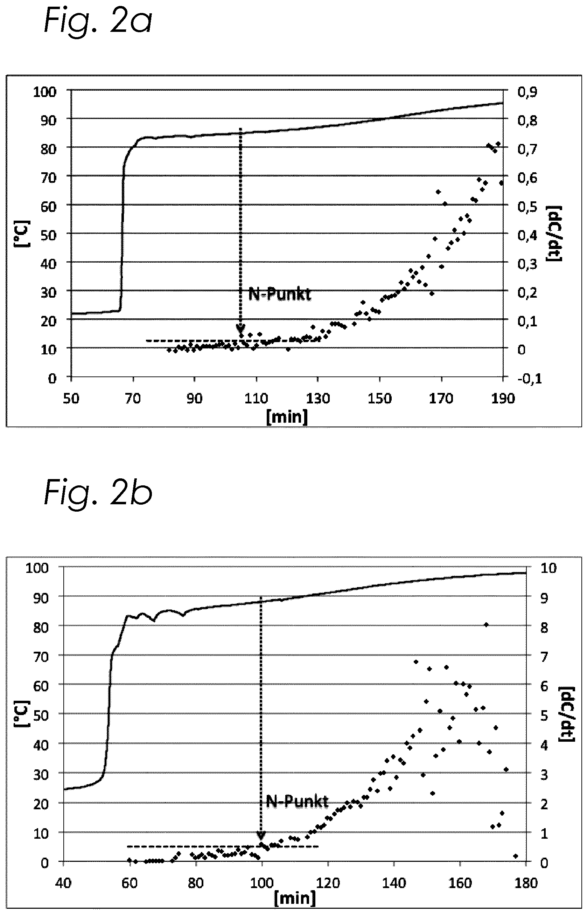

Aus den Fign. 2a, 2b wird deutlich, dass mittels Leitfähigkeit ein Umstellen von Mittellauf auf Nachlauf (Anstiegsänderung) möglich ist, jedoch nicht mit einem fixen Temperaturschaltpunkt im Geistrohr.

Die Fig. 2a, 2b zeigen zwei Beispiele kontinuierlicher Leitfähigkeitsmessung, dargestellt als Steigung der Leitfähigkeit dC/dt; Eine signifikante Änderung der Steigung (= N-Punkt) wird durch erstmaliges Überschreiten der zuvor ermittelten statistischen Schwankungsbreite (Mittelwert + 3x Standardabweichung) der Steigungswerte zu Beginn des Mittellaufs angezeigt.

Fig. 2a zeigt Hollermaische mit 3 Vol% EtOH à N-Punkt bei 88°C, Fig. 2b Apfelmaische mit 5 Vol% EtOH à N-Punkt bei 85°C.

### Liste der Bezugsziffern

- 1: Destillationsanlage
- 10: Auslauf
- 11: Kühler (Kondensator)
- 12: Leitfähigkeitssonde
- 13: Helm
- 14: Brennblase
- 15: Heizung
- 16: Vorlage
- 17: Dreiwegeventil

## Patentansprüche

1. Verfahren zur Bestimmung des Nachlaufabtrennpunktes (N) bei der Herstellung und/oder Reinigung von alkoholischen Getränken oder Extrakten mittels einer Destillationsanlage,
**dadurch gekennzeichnet, dass**
der Nachlaufabtrennpunkt (N) anhand einer kontinuierlichen Messung und Auswertung der elektrischen Leitfähigkeit des Destillats mittels mindestens einer Leitfähigkeitssonde (12) bestimmt wird, wobei zur Gewährleistung einer automatischen Umschaltung von Mittellauf auf Nachlauf ein mathematischer Algorithmus angewendet wird, welcher in einem Steuergerät bei einem Abweichungswert von der geringsten während des Mittellaufs gemessenen Leitfähigkeit oder bei einer Änderung der Steigung der Leitfähigkeit dC/dt die Vorlage von Mittellauf auf Nachlauf mechanisch wechselt.

2. Verfahren nach Anspruch 1,
**dadurch gekennzeichnet, dass**
die Messung der elektrischen Leitfähigkeit des Destillats im Auslauf (10) des Kühlers (11) der Destillationsanlage (1) erfolgt.

3. Verfahren zur Verarbeitung des nach Anspruch 1 oder 2 ermittelten Nachlaufabtrennpunktes (N),
**dadurch gekennzeichnet, dass**
das elektrische Signal einer Leitfähigkeitssonde (12) dergestalt verarbeitet wird, dass eine definierte Erhöhung der elektrischen Leitfähigkeit des Destillats zur Basis eines definierten im Mittellauf gemessenen Werts ermittelt und zum - vorzugsweise mechanischen - Umschalten der Vorlage (16) von Mittellauf auf Nachlauf genutzt wird.

4. Verfahren zur Verarbeitung des Nachlaufabtrennpunktes (N) nach Anspruch 3,
**dadurch gekennzeichnet, dass**
die definierte Erhöhung der elektrischen Leitfähigkeit des Destillats eine geringfügige Erhöhung der elektrischen Leitfähigkeit des Destillats zur Basis des niedrigsten im Mittellauf gemessenen Werts ist.

5. Verfahren zur Verarbeitung des Nachlaufabtrennpunktes (N) nach Anspruch 3 oder 4,
**dadurch gekennzeichnet, dass**
die Umschaltung der Vorlage (16) von Mittellauf auf Nachlauf über eine Umlenkung des Destillats beim Austritt aus der Vorlage erfolgt.

6. Verfahren nach Anspruch 5,
**dadurch gekennzeichnet, dass**
die Umlenkung über eine elektrische oder pneumatische Ansteuerung eines Mehrwegeventils erfolgt.

7. Verfahren nach Anspruch 5,
**dadurch gekennzeichnet, dass**
die Umlenkung durch Drehung der gesamten Vorlage erfolgt.

## Claims

1. A method for determining the final run separation point (N) when producing and/or purifying alcoholic beverages or extracts by means of a distillation unit, **characterized in that**
the final run separation point (N) is determined by means of continuously measuring and evaluating the electrical conductivity of the distillate by means of at least one conductivity probe (12), wherein a mathematical algorithm is used for ensuring automatic switching over from middle run to final run, said algorithm mechanically changing over the main tank from middle run to final run in a control device for a deviation value of the least conductivity measured during the middle run, or for a change in the slope of the conductivity dC/dt.

2. The method according to claim 1,
**characterized in that**
the measuring of the electrical conductivity of the distillate takes place in the outlet (10) of the cooler (11) of the distillation unit (1).

3. A method for processing the final run separation point (N) determined according to claim 1 or 2,
**characterized in that**
the electrical signal of a conductivity probe (12) is processed such that a defined increase in the electrical conductivity of the distillate is determined relative to the basis of a defined value measured in the middle run and used for preferably mechanically switching over the main tank (16) from middle run to final run.

4. The method for processing the final run separation point (N) according to claim 3,
**characterized in that**
the defined increase in the electrical conductivity of the distillate is a slight increase in the electrical conductivity of the distillate relative to the basis of the least value measured in the middle run.

5. The method for processing the final run separation point (N) according to claim 3 or 4,
**characterized in that**
the switching over of the main tank (16) from middle run to final run takes place by means of diverting the distillate when exiting the main tank.

6. The method according to claim 5,
**characterized in that**
the diverting takes place by means of an electrical or pneumatic actuating of a multi-way valve.

7. The method according to claim 5,
**characterized in that**
the diverting takes place by rotating the entire main tank.

## Revendications

1. Procédé de détermination du temps de séparation de la queue de distillation (N) lors de la fabrication et/ou de la purification de boissons alcooliques ou d'extraits au moyen d'une installation de distillation,
**caractérisé en ce que**
le temps de séparation de la queue de distillation (N) est déterminé à l'aide d'une mesure et d'une évaluation continues de la conductivité électrique du distillat au moyen d'au moins une sonde de conductivité (12), dans lequel, afin de garantir une commutation automatique du coeur de distillation à la queue de distillation, un algorithme mathématique est appliqué qui dans un appareil de commande, dans le cas d'une valeur d'écart par rapport à la plus petite conductivité mesurée pendant le coeur de distillation ou dans le cas d'une variation de la pente de la conductivité dC/dt, fait passer mécaniquement la tête de distillation du coeur de distillations à la queue de distillation.

2. Procédé selon la revendication 1,
**caractérisé en ce que**
la mesure de la conductivité électrique du distillat est effectuée dans la sortie (10) du condenseur (11) de l'installation de distillation (1).

3. Procédé de traitement du temps de séparation de la queue de distillation (N), établi selon la revendication 1 ou 2,
**caractérisé en ce que**
le signal électrique d'une sonde de conductivité (12) est traité de telle sorte qu'une augmentation définie de la conductivité électrique du distillat est établie par rapport à la base d'une valeur définie, mesurée dans le coeur de distillation, et est utilisée pour la commutation - de préférence mécanique - de la tête de distillation (16) du coeur de distillation à la queue de distillation.

4. Procédé de traitement du temps de séparation de la queue de distillation (N) selon la revendication 3,
**caractérisé en ce que**
l'augmentation définie de la conductivité électrique du distillat est une faible augmentation de la conductivité électrique du distillat par rapport à la base de la valeur la plus basse mesurée dans le coeur de distillation.

5. Procédé de traitement du temps de séparation de la queue de distillation (N) selon la revendication 3 ou 4,
**caractérisé en ce que**
la commutation de la tête de distillation (16) du coeur de distillation à la queue de distillation est effectuée par une déviation du distillat lors de la sortie de la tête de distillation.

6. Procédé selon la revendication 5,
**caractérisé en ce que**
la déviation est effectuée par un pilotage électrique ou pneumatique d'une vanne à voies multiples.

7. Procédé selon la revendication 5,
**caractérisé en ce que**
la déviation est effectuée par le pivotement de toute la tête de distillation.
